# EUROPEAN PATENT APPLICATION

(11) **EP 4 000 741 A1**
(43) Date of publication of application: **25.05.2022**
(21) Application number: 21208023.8
(22) Date of filing: 12.11.2021
(51) Int. Cl.: B05B 1/00, B05B 5/00

(54) **ELECTRIC SPRAYER**

(30) Priority: 13.11.2020 US 202063113377 P
(71) Applicant: Techtronic Cordless GP, Anderson, SC 29621 (US)
(72) Inventor: MOYLAN, Justin, Easley, SC 29642 (US); TONER, Justin, Anderson, SC 29621 (US); KANE, Adam, Anderson, SC 29621 (US); SUAREZ, Josef Manuel, Anderson, SC 29621 (US)
(74) Representative: Parker, Andrew James

(57) **Abstract**

A fluid sprayer includes a housing defining a reservoir attachment portion, a pump supported within the housing, a nozzle assembly supported by the housing and located in fluid communication with the pump, and an electrostatic charging circuit configured to impart an electrostatic charge in a fluid pumped out of the nozzle assembly by the pump. The fluid sprayer also includes a first reservoir including a first latch configured to releasably secure the first reservoir to the reservoir attachment portion to fluidly couple the first reservoir to the pump. The fluid sprayer further includes a second reservoir including a tank, an adapter, and a hose fluidly connecting the tank to the adapter, the adapter including a second latch configured to releasably secure the adapter to the reservoir attachment portion to fluidly couple the tank to the pump.

## Description

### FIELD OF THE DISCLOSURE

The disclosure relates to fluid delivery devices, and more particularly to portable, battery-powered, electrostatic liquid sprayers.

### BACKGROUND OF THE DISCLOSURE

Powered sprayers, such as foggers, misters, and the like, are commonly used to disperse liquid solutions onto surfaces in vapor, mist or fog form. Some sprayers include electrostatic charging systems to electrostatically charge the spray droplets of the liquid solution, causing the droplets to cling to the targeted surfaces.

### SUMMARY OF THE DISCLOSURE

The present disclosure provides, in one aspect, a fluid sprayer including a housing defining a reservoir attachment portion, a pump supported within the housing, a nozzle assembly supported by the housing and located in fluid communication with the pump, and an electrostatic charging circuit configured to impart an electrostatic charge in a fluid pumped out of the nozzle assembly by the pump. The fluid sprayer also includes a first reservoir including a first latch configured to releasably secure the first reservoir to the reservoir attachment portion to fluidly couple the first reservoir to the pump. The fluid sprayer further includes a second reservoir including a tank, an adapter, and a hose fluidly connecting the tank to the adapter, the adapter including a second latch configured to releasably secure the adapter to the reservoir attachment portion to fluidly couple the tank to the pump.

The present disclosure provides, in another aspect, a fluid sprayer including a housing defining a reservoir attachment portion having an alignment recess, a pump supported within the housing, a nozzle assembly supported by the housing and located in fluid communication with the pump, and an electrostatic charging circuit configured to induce an electrostatic charge in a fluid pumped out of the nozzle assembly by the pump. The fluid sprayer also includes a first reservoir configured to releasably secure to the reservoir attachment portion and including a first alignment ridge configured to be received into the alignment recess to align the first reservoir with the reservoir attachment portion. The fluid sprayer further includes a second reservoir including a tank, an adapter configured to releasably secure to the reservoir attachment portion to fluidly couple the tank to the pump, and a hose fluidly connecting the tank to the adapter, the adapter including a second alignment ridge configured to be received into the alignment recess to align the adapter with the reservoir attachment portion.

The present disclosure provides, in yet another aspect, a fluid sprayer including a housing defining a reservoir attachment portion, a pump supported within the housing, a nozzle assembly supported by the housing and located in fluid communication with the pump, and an electrostatic charging circuit configured to induce an electrostatic charge in a fluid pumped out of the nozzle assembly by the pump. The fluid sprayer also includes a first reservoir configured to releasably secure to the reservoir attachment portion to fluidly couple the first reservoir to the pump, the first reservoir including a first alignment ridge configured to align the first reservoir in a first orientation relative to the reservoir attachment portion and in a second orientation relative to the reservoir attachment portion, the second orientation being one hundred eighty degrees away from the first orientation. The fluid sprayer further includes a second reservoir including a tank, an adapter configured to releasably secure to the reservoir attachment portion to fluidly couple the tank to the pump, and a hose fluidly connecting the tank to the adapter, the adapter including a second alignment ridge configured to align the adapter in a third orientation relative to the reservoir attachment portion and in a fourth orientation relative to the reservoir attachment portion, the third orientation being one hundred eighty degrees away from the fourth orientation.

Other aspects of the disclosure will become apparent by consideration of the detailed description and accompanying drawings.

### BRIEF DESCRIPTION OF THE DRAWINGS

FIG. 1 is a perspective view of a portable, battery-powered liquid sprayer according to an embodiment of the disclosure.
FIG. 2 is another perspective view of the sprayer of FIG. 1.
FIG. 3 is another perspective view of the sprayer of FIG. 1 with portions removed.
FIG. 4 is a perspective view of an adjustable nozzle assembly of the sprayer of FIG. 1.
FIG. 5 is a perspective cross-sectional view of the adjustable nozzle assembly of FIG. 4, taken along line 5-5 of FIG. 4.
FIGS. 6A-6D are perspective views of the adjustable nozzle assembly of FIG. 4 adjusted to different positions.
FIG. 7 is an exploded perspective view of the adjustable nozzle assembly of FIG. 4.
FIG. 8 is a schematic view of an electrostatic charging circuit of the sprayer of FIG. 1.
FIG. 9 is an exploded perspective view of a high voltage electrode assembly of the electrostatic charging circuit of FIG. 8.
FIGS. 10A and 10B are perspective views of a grounding ring of the electrostatic charging circuit of FIG. 8.
FIG. 11 is a perspective view of a grounding ring according to another embodiment.
FIG. 12 is a perspective view of a grounding ring according to yet another embodiment.
FIG. 13 is a perspective view of a grounding ring according to yet another embodiment.
FIGS. 14A-14C are perspective views of a grounding ring according to yet another embodiment.
FIG. 15 is a schematic view of a liquid recovery system of the sprayer of FIG. 1.
FIGS. 16A and 16B are perspective views of portions of the sprayer of FIG. 1, illustrating channels of the liquid recovery system.
FIG. 17 is a schematic view of the sprayer of FIG. 1 further including a removable nozzle assembly and a safety switch.
FIGS. 18A and 18B are partially exploded perspective views of the sprayer of FIG. 1.
FIGS. 19 is partially exploded perspective view of a portion of the sprayer of FIG. 1.
FIG. 20 is a partial cross-sectional view of the sprayer of FIG. 1, taken along line 20-20 of FIG. 2.
FIG. 21 is a partially exploded perspective view of a portion of a reservoir cap of the sprayer of FIG. 1.
FIG. 22 is a perspective view of a portion of the sprayer of FIG. 1, illustrating a nozzle storage receptacle.
FIG. 23 is a perspective view of a portion of the sprayer of FIG. 1, illustrating a nozzle storage receptacle according to another embodiment.
FIGS. 24A and 24B are perspective views of a remote reservoir that can be utilized with the sprayer of FIG. 1.
FIG. 25A is perspective view of portions of the sprayer of FIG. 1, showing an onboard reservoir.
FIG. 25B is a schematic view of portions of the sprayer of FIG. 1, showing an onboard reservoir according to another embodiment.
FIG. 25C is a perspective view of an adapter for coupling the remote reservoir of FIG. 24A to the sprayer of FIG. 1.
FIG. 26 is a schematic view illustrating a priming and recirculating system of the sprayer of FIG. 1.
FIGS. 27A-27D are side views of the sprayer of FIG. 1.

Before any embodiments of the disclosure are explained in detail, it is to be understood that the disclosure is not limited in its application to the details of embodiment and the arrangement of components set forth in the following description or illustrated in the following drawings. The disclosure is capable of other embodiments and of being practiced or of being carried out in various ways. Also, it is to be understood that the phraseology and terminology used herein is for the purpose of description and should not be regarded as limiting.

### DETAILED DESCRIPTION

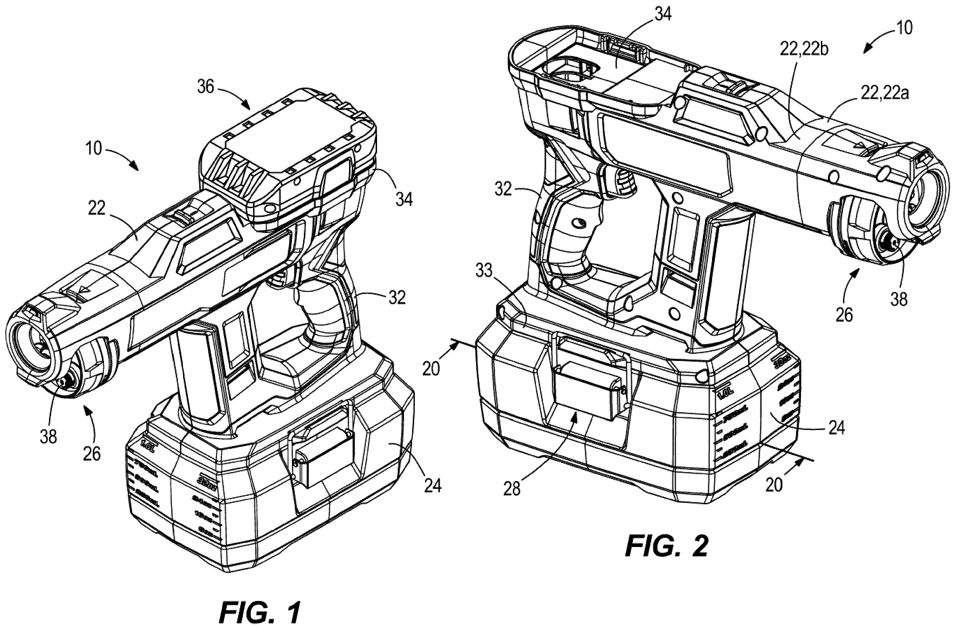

FIGS. 1 and 2 illustrate a portable, battery-powered, electrostatic liquid sprayer 10 according to an embodiment of the present disclosure. The sprayer 10 includes a housing 22, a reservoir 24 to hold a liquid solution (e.g., a disinfecting solution), and a nozzle assembly 26 to dispense the solution as an atomized spray. The housing 22 is formed from a pair of clamshell housing halves 22a, 22b. The reservoir 24 is removably coupled to the housing 22 via latches 28. The housing 22 defines a handle portion 32, a reservoir attachment portion 33 that removably couples to the reservoir 24, and a battery receptacle 34 that selectively couples to a removable and rechargeable battery pack 36 to provide DC electrical power to the sprayer 10. In alternative embodiments, the sprayer 10 may be AC powered (e.g., plugged into a standard home electrical socket), gas-powered (e.g., by one or more internal combustion engines), and the like. The handle portion 32 may be grasped by the user during use to manipulate the orientation and position of the sprayer 10. As will be discussed in further detail below, the nozzle assembly 26 is adjustable to enable switching between different removable nozzles 38 that impart different spray characteristics to the spray (e.g., flow rate, spray pattern, spray droplet size, etc.).

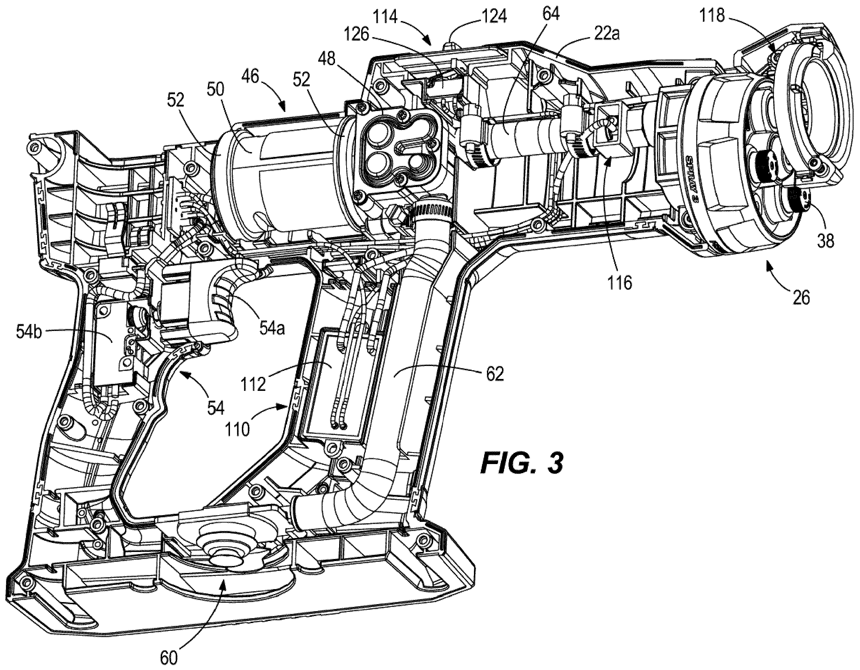

With reference to FIG. 3, the sprayer 10 includes a motor and pump assembly 46 that includes a pump 48 in fluid communication with the reservoir 24 and with the nozzle assembly 26, and a motor 50 configured to drive the pump 48. The motor and pump assembly 46 is supported within the main housing 40 by damping elements 52 positioned between the motor and pump assembly 46 and walls of the housing 22 to dampen transmission of vibration therebetween. The handle portion 32 of the housing 22 supports a trigger assembly 54 with a trigger 54a that actuates a trigger switch 54b to selectively activate the motor 50, causing the pump 48 to draw liquid solution from the reservoir 24 and pump the solution toward the nozzle assembly 26 to produce the atomized spray. In the illustrated embodiment, the pump 48 is a diaphragm pump. In other embodiments, other types of pumps may be utilized (e.g., a piston pump, a gear pump, a peristaltic pump, and the like). The sprayer 10 also includes a solution inlet 60 located at the reservoir attachment portion 33 and configured to fluidly connect to the reservoir 24, an inlet conduit 62 fluidly connecting the solution inlet 60 to an inlet side of the pump 48, and an outlet conduit 64 fluidly connected to an outlet side of the pump 48 to direct the liquid solution exiting the pump 48 toward the nozzle assembly 26.

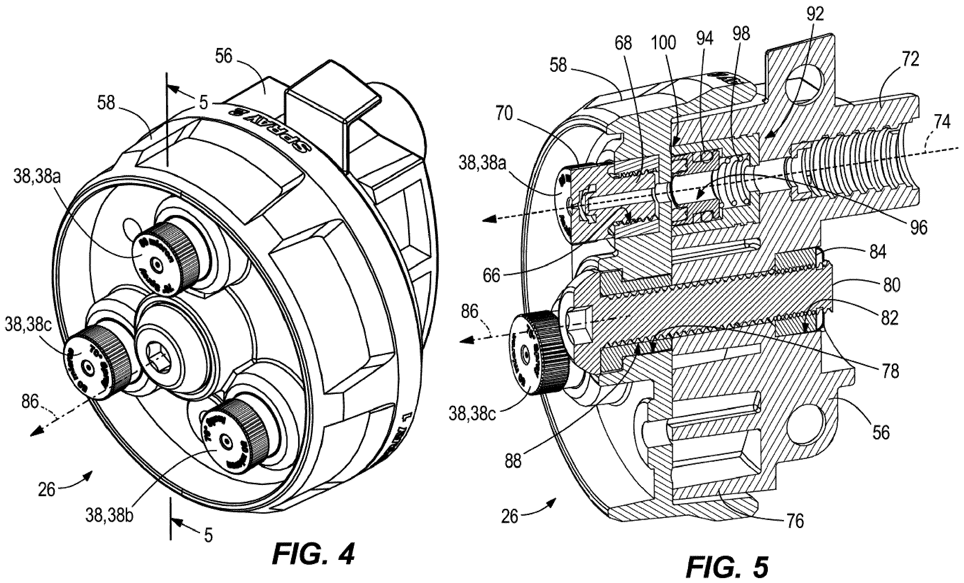

With reference to FIGS. 4-5, the adjustable nozzle assembly 26 includes a nozzle mount 56 fixedly supported by the sprayer housing 22, and a removable and rotatable selection wheel 58 removably coupled to the nozzle mount 56. In the embodiment shown in FIG. 4, the selection wheel 58 supports multiple nozzles 38 including a first nozzle 38a, a second nozzle 38b, and a third nozzle 38c each configured to impart different spray characteristics to the solution sprayed out from the respective nozzle 38a-38c. In the illustrated embodiment, the different nozzles 38a-38c correspond to different droplet sizes of the atomized solution sprayed from the nozzle, including a smallest droplet spray, a medium droplet spray, and a largest droplet spray, respectively. However, the nozzles 38a-38c may be configured to vary other parameters of the spray instead of or in addition to the droplet size, such as the spray pattern, the flow rate, the spray velocity, and the like. Although three nozzles 38 are illustrated, it should be appreciated that the selection wheel 58 may be configured to support fewer than three nozzles (e.g., two nozzles), or more than three nozzles (e.g., four nozzles, five nozzles, etc.) in a manner similar to that described herein.

In the illustrated embodiment, the selection wheel 58 defines three threaded nozzle apertures 66 that each receive a respective nozzle 38 by threaded engagement therewith. Specifically, each nozzle 38 includes a threaded inlet portion 68 that tightens into a respective threaded nozzle aperture 66, and a gripping portion 70 that may be grasped by the user to twist the nozzle 38 to thereby tighten and secure the nozzle 38 to the selection wheel 58, or loosen and remove the nozzle 38 therefrom. By providing a threaded engagement between the nozzles 38 and the selection wheel 58, and by providing a gripping portion 70, each nozzle 38 can be installed or removed by hand, without the use of tools. This enables the user to easily remove the nozzles for, e.g., maintenance, cleaning, or for switching with different nozzles to achieve a spray with a wide variety of unique desired characteristics.

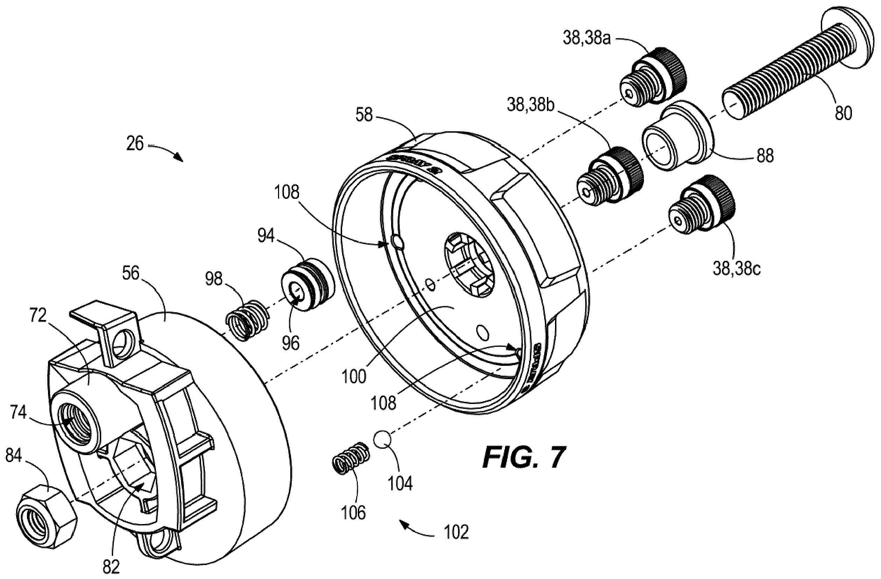

With reference to FIGS. 5 and 7, the nozzle mount 56 includes a nozzle mount inlet 72 that defines an inlet passageway 74 by which liquid solution enters the nozzle assembly 26. The nozzle mount 56 also includes a wheel hub portion 76 that defines a mounting aperture 78 for receiving a fastener, such as threaded screw 80, to rotatably couple the selection wheel 58 to the nozzle mount 56. In the illustrated embodiment, the wheel hub portion 76 also defines a hexagonal recess 82 that receives a nut 84 to which the screw 80 fastens. The screw 80 can be loosened to remove the selection wheel 58 from the nozzle mount 56 (e.g., for cleaning or maintenance). The selection wheel 58 is rotatable relative to the nozzle mount 56 about an axis 86 defined by the screw 80, to enable switching between different nozzle positions corresponding to the different nozzles 38a-38c, and to a priming position for priming the pump 48, as will be described below. Accordingly, the selection wheel 58 also includes a journal bearing 88 that interfaces with the screw 80 to facilitate smooth rotation between the various positions of the selection wheel 58.

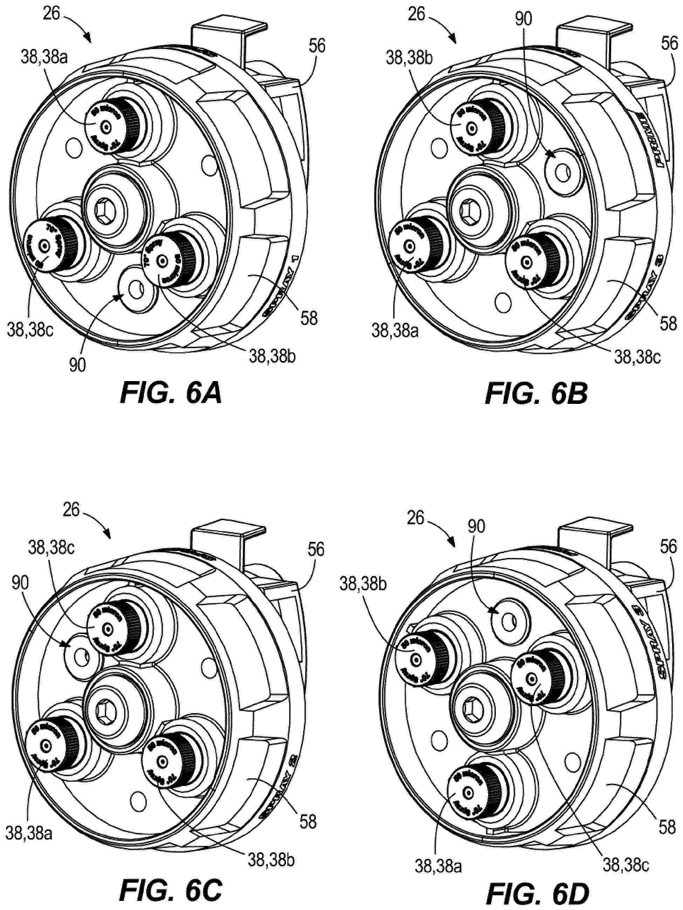

With reference to FIGS. 6A-6D, the selection wheel 58 is rotatable between different angular positions corresponding to different selections of the nozzles 38a-38c, or to the priming position for evacuating air which may come to occupy the fluid pathways between the reservoir 24 and the nozzle assembly 26 (e.g., due to periods of non-use). FIG. 6A illustrates the selection wheel 58 in a first position or smallest droplet position at which the liquid solution flows out from the first nozzle 38a. FIG. 6B illustrates the selection wheel 58 in a second position or medium droplet position at which the liquid solution flows out from the second nozzle 38b. FIG. 6C illustrates the selection wheel 58 in a third position or largest droplet position at which the liquid solution flows out from the third nozzle 38c. FIG. 6D illustrates the selection wheel 58 located in a fourth position or priming position, in which a priming aperture 90 formed in the selection wheel 58 is aligned with the flow passageway extending through the nozzle mount 56. Since the nozzles 38 include relatively small outlet openings for ejecting the liquid solution, air which may become trapped within the fluid passageways of the sprayer 10 (e.g., the inlet passageway 74, the outlet conduit 64, the pump 48, etc.) may take a long time to evacuate through the nozzles 38 after periods of non-use. The selection wheel 58 thus includes the priming aperture 90 that is substantially larger than the nozzle openings of the nozzles 38, and provides a smaller pressure drop and less restricted flow that is desirable for priming the pump 48 and associated fluid passageways. When operating the sprayer 10, the user can rotate the selection wheel 58 to adjust between the first, second, third positions to select different nozzles 38a-38c according to the particular spray characteristics desired. And after periods of non-use, the user can rotate the selection wheel 58 to the fourth position and pull the trigger 54a to activate the pump 48, thereby evacuating any trapped air and priming the sprayer 10 for use.

Referring again to FIGS. 5 and 7, the nozzle mount 56 also includes a pressure seal assembly 92 that maintains a liquid-tight seal between the inlet passageway 74 and the selection wheel 58. The pressure seal assembly 92 generally resides within an end portion of the inlet passageway 74 located proximate the selection wheel 58. The pressure seal assembly 92 includes a pressure seal member 94 having a central bore 96 through which the liquid solution is permitted to flow, and a pressure seal spring 98 that biases the pressure seal member 94 forwardly toward the selection wheel 58. When the selection wheel 58 is rotated between the first, second, third, and fourth positions, the pressure seal member 94 contacts a rear surface 100 of the selection wheel 58 to create a liquid-tight seal between the selection wheel 58 and the inlet passageway 74. Accordingly, the pressure seal assembly 92 prevents liquid solution from leaking from the nozzle assembly 26 as the selection wheel 58 is located at the first, second, third, or fourth positions. As the selection wheel 58 is rotated between positions, the pressure seal member 94 maintains contact with the rear surface 100 to inhibit leakage of liquid solution, although the pressure seal member 94 may not fully seal against the rear surface 100 during adjustment.

With reference to FIG. 7, the adjustable nozzle assembly 26 also includes a detent assembly 102 that releasably arrests rotation of the selection wheel 58 at each of the four rotational positions shown in FIGS. 6A-6D (i.e., the smallest droplet position, the medium droplet position, the largest droplet position, and the priming position). The detent assembly 102 includes a ball 104 and a ball spring 106 located within a recess (not shown) formed in the nozzle mount 56. The selection wheel 58 includes depressions 108 formed in the rear surface 100 and located at angular positions that correspond to the first, second, third, and fourth positions of the selection wheel 58. The ball spring 106 biases the ball 104 toward the selection wheel 58, and as the selection wheel 58 is rotated to each of the four positions shown in FIGS. 6A-6D, the ball 104 engages each respective depression 108 to releasably arrest rotation of the selection wheel 58 at the respective position. When the user applies sufficient torque to the selection wheel 58, the biasing force of the spring 106 is overcome and the ball 104 disengages from the respective depression 108, allowing the selection wheel 58 to be rotated to another position.

In operation, the user adjusts the selection wheel 58 to the first, second, or third position as desired, and depresses the trigger 54a to activate the motor 50. The motor 50 drives the pump 48 to begin pumping liquid solution from the reservoir 24 toward the adjustable nozzle assembly 26. The solution moving from the pump 48 enters the inlet conduit 62, flows through the pressure seal assembly 92, and sprays outward from the selected nozzle 38a-38c. To select a different nozzle 38a-38c, the user releases the trigger 54a, rotates the selection wheel 58 to another of the first, second, or third positions as desired. To prime the sprayer 10, e.g., to remove air from the passageways after periods of non-use, the user rotates the selection wheel 58 to the fourth position, i.e., the priming position, and depresses the trigger 54a to activate the motor 50. The pump 48 will then force any trapped air out of the fluid passageways and out through the priming aperture 90.

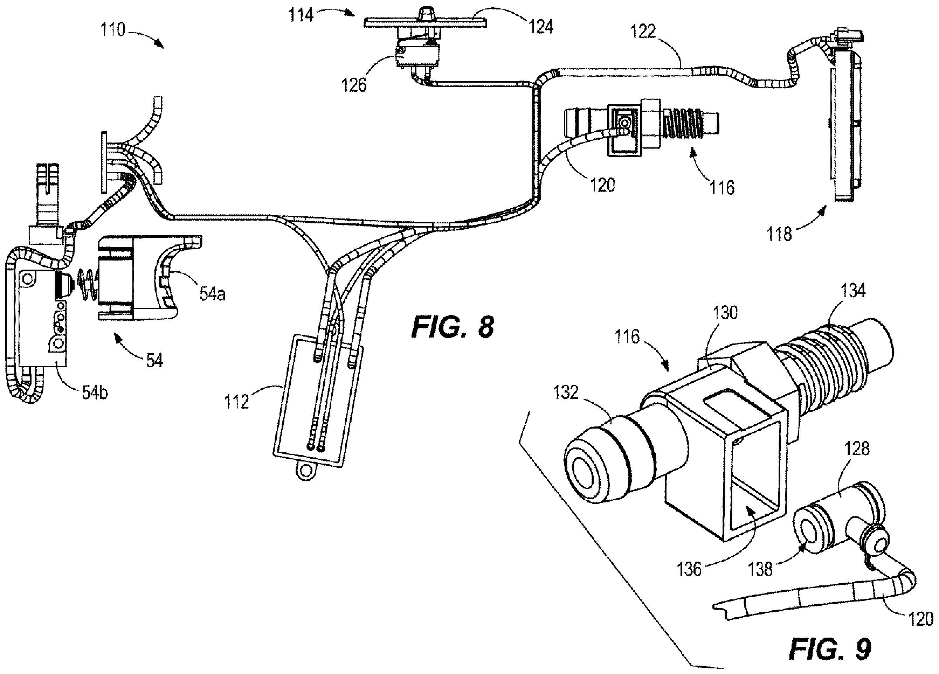

With reference to FIGS. 3 and 8, the sprayer 10 also includes an electrostatic charging circuit 110 operable to impart an electrostatic charge in the atomized liquid droplets exiting the nozzle assembly 26 during operation of the sprayer 10. The electrostatic charging circuit 110 includes a high voltage supply module 112 supported within the housing 22, a charging on/off switch assembly 114, a first or high voltage electrode assembly 116, and a second electrode assembly or grounding ring assembly 118. The high voltage supply module 112 receives power from the battery pack 36 and supplies power, via a high voltage wire 120, to the high voltage electrode assembly 116. The grounding ring assembly 118 is electrically coupled to the high voltage supply module 112 via a grounding wire 122 to complete the charging circuit 110.

The charging on/off switch assembly 114 includes a slider 124 movable between on and off positions to actuate a charging on/off switch 126. In the illustrated embodiment of the sprayer 10, the motor 50 is electrically connected to an output terminal of the trigger switch 54b, so that when the trigger 54a is actuated, the motor 50 is activated to begin pumping the liquid solution toward the nozzle assembly 26. The charging on/off switch 126 is located between the output terminal of the trigger switch 54b and the high voltage supply module 112. Accordingly, when the trigger 54a is released such that the trigger switch 54b is open, the charging circuit 110 is necessarily deactivated. When the trigger 54a is pulled to close the trigger switch 54b, the electrostatic charging circuit 110 can then be toggled on or off via actuation of the charging on/off switch assembly 114. The sprayer 10 can thus be operated with the electrostatic charging circuit 110 enabled or disabled as desired by the user by moving the slider 124 of the charging on/off switch assembly between the on and off positions. It should be understood that in other embodiments of the sprayer 10 (not shown), the charging on/off switch assembly 114 may be omitted, so that actuation of the trigger switch 54b activates both the motor 50 and the electrostatic charging circuit 110.

With reference to FIG. 9, the high voltage electrode assembly 116 includes a high voltage electrode 128 and an electrode housing 130 with an electrode housing inlet 132, an electrode housing outlet 134, and an electrode receptacle 136. In the illustrated embodiment, the electrode housing inlet 132 couples to the outlet conduit 64 to receive liquid solution from the pump 48, and the electrode housing outlet 134 couples to the nozzle mount inlet 72. In other embodiments (not shown), the high voltage electrode assembly 116 can be located at other points along a continuous flow path between the reservoir 24 and the nozzle assembly 26, including, e.g., upstream from the pump 48. In the illustrated embodiment, the high voltage electrode 128 is provided as a conductive tube formed from a conductive material such as, e.g., copper, brass, or the like. The high voltage electrode 128 is held and sealed within the electrode receptacle 136 by potting (not shown), and defines a central passageway 138 through which the liquid solution flows between the electrode housing inlet and outlet 132, 134. During operation of the sprayer 10, the liquid solution thus passes through the central passageway 138 of the high voltage electrode 128 such that the liquid solution is directly charged by direct contact with the high voltage electrode 128.

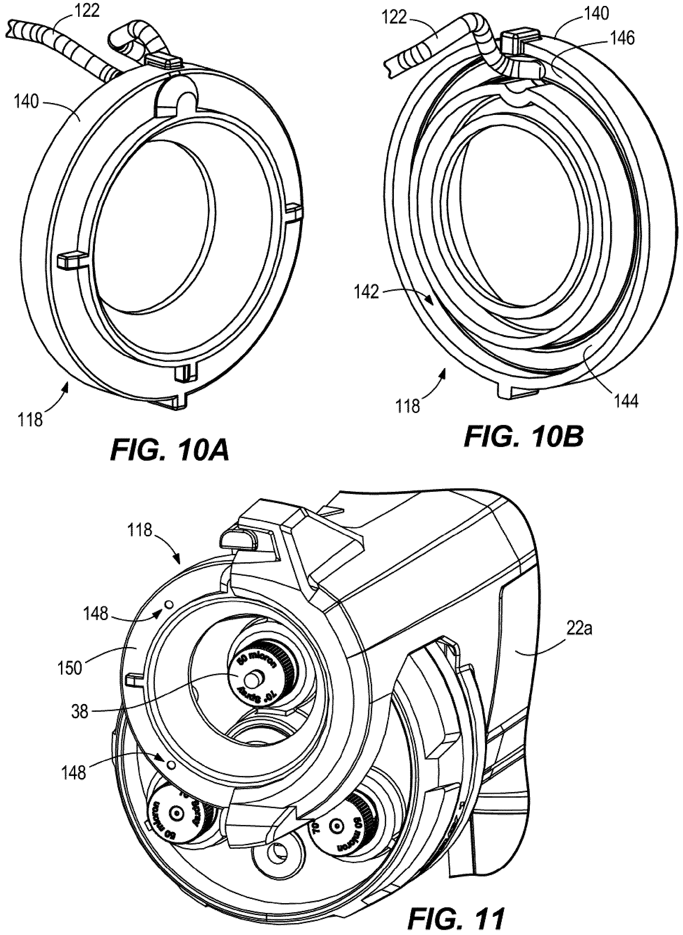

FIGS. 10A-14C illustrate embodiments of the grounding ring assembly 118. In one embodiment shown in FIGS. 10A and 10B, the grounding ring assembly 118 includes an annular ring holder 140 formed from an insulating material (e.g., plastic) and having an annular groove 142 that receives an electrode ring 144 formed as a ring-shaped PCB having a copper trace 146. The grounding wire 122 is soldered to the trace 146 at an uppermost portion of the electrode ring 144. As shown in FIG. 3, the grounding ring assembly 118 is supported by the housing 22 at a location forward of the nozzle assembly 26 and centered about the spray nozzle 38. When the charging circuit 110 is active, an electric field develops between the grounding ring assembly 118 and any conductive material (such as, e.g., the liquid solution) in electrical contact with the high voltage electrode 128. As the atomized spray droplets of the liquid solution exit the spray nozzle 38, the droplets pass through the interior of the grounding ring assembly 118 and through the electric field, thereby acquiring an electrostatic charge. The acquired charge enables the spray droplets to better adhere or "stick" to targeted surfaces. A diameter of the electrode ring 144 is minimized to maximize the electrostatic charging effect, without interfering with the spray cone emitted from the nozzle assembly 26.

In another embodiment of the grounding ring assembly 118 shown in FIG 11, the ring holder 140 includes a plurality of apertures 148 (e.g., four apertures) provided in a forward face 150 of the ring holder 140 to expose the copper trace 146 at multiple locations about the ring holder 140. The apertures 148 provide pathways to expose the copper trace 146 to the air and the moist environment surrounding the grounding ring assembly 118, to promote the development of the electric field when the charging circuit 110 is active during operation of the sprayer 10.

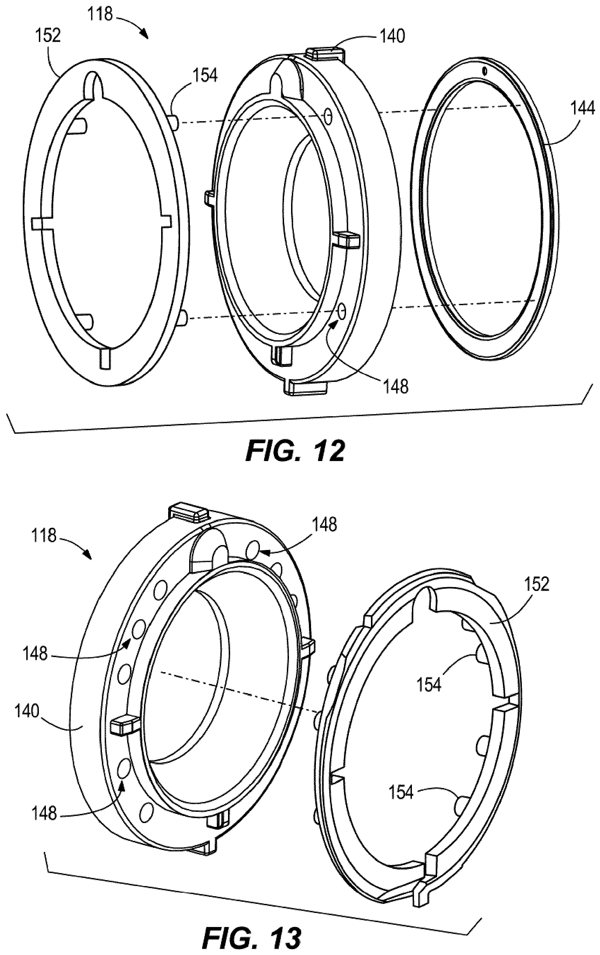

In another embodiment of the grounding ring assembly 118 shown in FIG. 12, the ring holder 140 again includes the four apertures 148 formed in the forward face 150 thereof, and the grounding ring assembly 118 further includes a rubber washer 152 that is coupled to the forward face 150. The rubber washer 152 includes multiple pins 154 (e.g., four pins) that each extend into a corresponding aperture 148 in the ring holder 140 to fill the apertures 148. The rubber washer 152 provides some conductivity (i.e., the rubber material is more conductive than the plastic material of the ring holder 140) to enable formation of the electric field, while also preventing short circuiting of the current moving through the charging circuit 110 that could otherwise occur when the apertures 148 are unoccupied (e.g., due to moisture ingress). In other embodiments, the rubber washer 152 may be formed from materials other than rubber, including conductive materials, or insulating dielectrics.

In another embodiment the of grounding ring assembly 118 shown in FIG. 13, the ring holder 140 may include ten apertures 148 formed in the forward face 150 (other numbers of apertures are also possible), and the rubber washer 152 may include ten corresponding pins 154 to fill the ten apertures 148.

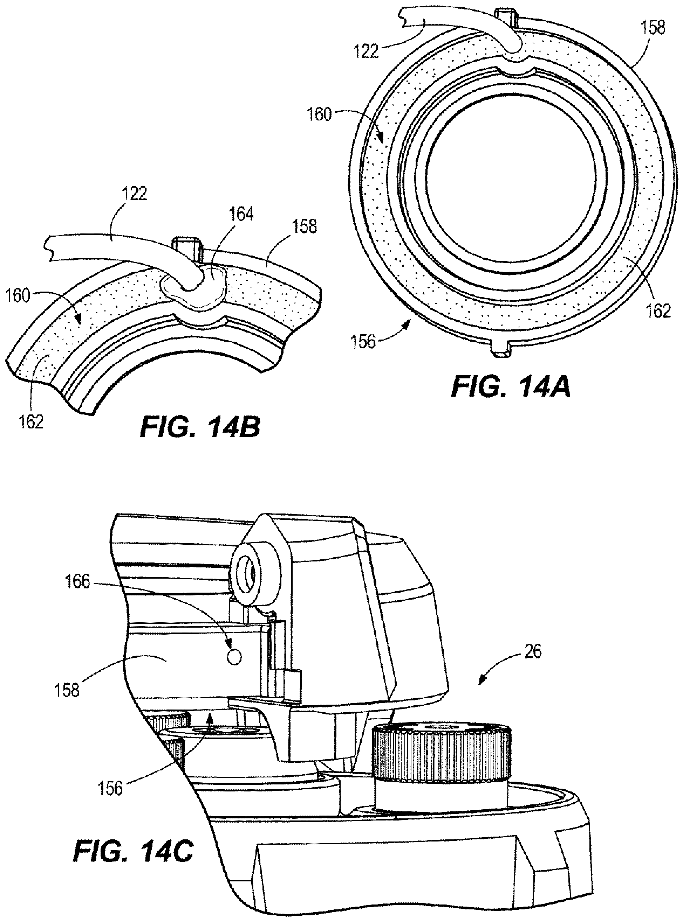

FIGS. 14A-14C illustrate an alternative embodiment of the grounding ring assembly 156 that includes an annular ring holder 158 formed from an insulating material (e.g., plastic) and having an annular groove 160 that receives an electrode ring (not shown) made from a conductive material (e.g., a metal such as brass). In some embodiments, the plastic forming the ring holder 158 can have some dielectric properties, or even conductive properties. The grounding wire 122 is soldered to the electrode ring, and an adhesive is provided inside the annular groove 160 to secure the electrode ring within the ring holder 158. A primer (not shown) is applied to both the ring holder 158 and the electrode ring, and a potting 162 is deposited into the groove 160 and over the electrode ring. The primer promotes adhesion between the potting 162 and the ring holder 158 to prevent ingress of moisture. A silicone glue 164 is applied to the potting 162 and the grounding wire 122 at the solder location to further insulate the solder connection. With reference to FIG. 14C, an aperture 166 is provided in the ring holder 158 at a location 180 degrees opposite from the location where the grounding wire 122 is soldered to the electrode ring. The aperture 166 exposes a portion of the grounding ring to the outside air and strengthens the electric field around the atomized droplets of the liquid solution to induce an electrostatic charge.

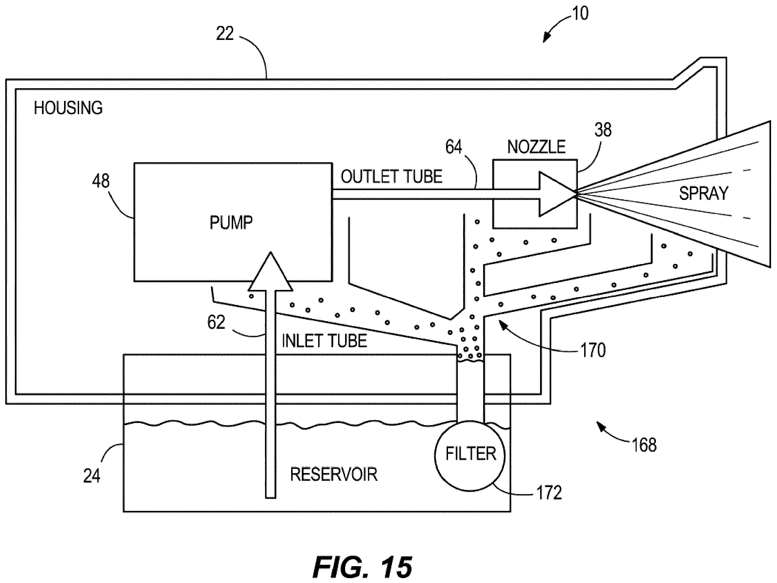

With reference to FIG. 15, the sprayer 10 can also include a liquid recovery system 168 for collecting any liquid solution that may leak from the components of the sprayer 10 during operation, or collecting electrostatically charged droplets that may be attracted back to the sprayer 10. The liquid recovery system 168 directs the collected liquid solution back into the reservoir 24. The liquid recovery system 168 can include one or more channels 170 provided in the housing 22 to direct the leaked liquid solution back into the reservoir 24. The liquid recovery system 168 can also include a filter 172 for filtering the collected liquid solution before it is returned into the reservoir 24 to prevent contaminants from entering the reservoir 24. The liquid solution can flow through the channels 170 by gravity, or the liquid recovery system 168 can further include a mechanical means (e.g., an auxiliary pump) to over the liquid solution through the channels 170 and back to the reservoir 24.

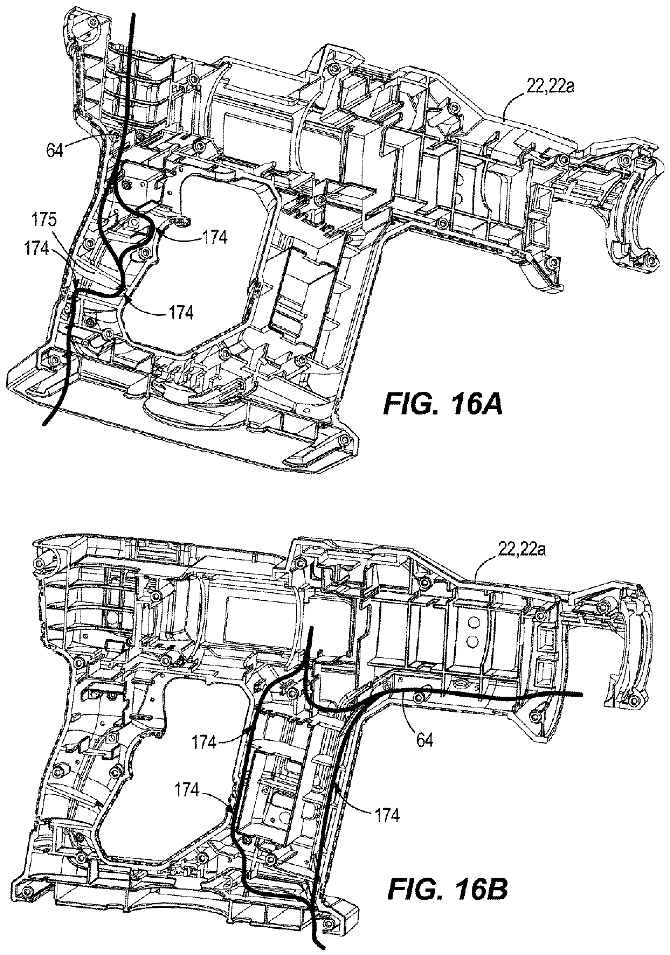

With reference to FIGS. 16A and 16B, the channels 170 of the liquid recovery system 168 can be provided in the form of notches 174 formed into internal baffles 175 provided within the clamshell housing halves 22a, 22b. The channels 170 are defined between successive notches 174 to allow liquid solution trapped within the housing 22 to drain, by gravity, toward the reservoir attachment portion 33 and toward the reservoir 24. The channels 170 guide any internally leaked liquid solution away from critical or sensitive components (e.g., such as high voltage supply module 112) and back to the reservoir 24.

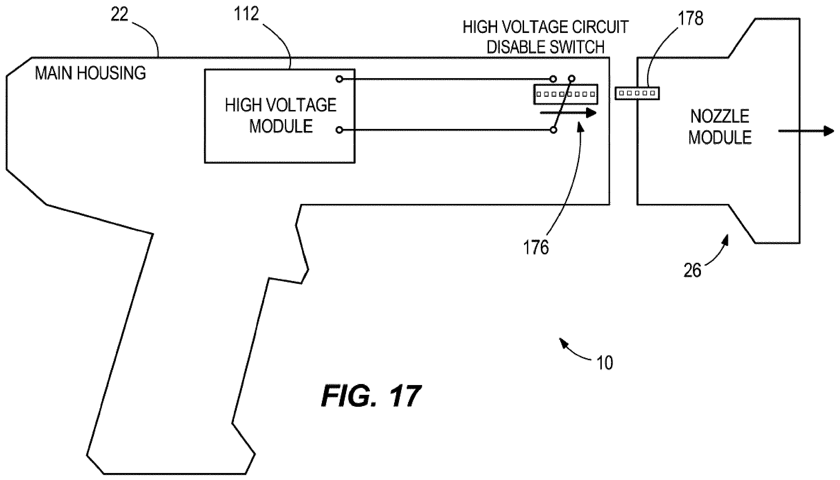

With reference to FIG. 17, in one embodiment of the sprayer 10, the nozzle assembly 26 may be provided as a removable module that is selectively removable from the housing 22 (e.g., to perform cleaning or maintenance of the nozzle assembly 26 or the housing 22). In this embodiment, the sprayer 10 may further be provided with a safety switch 176 that deactivates the charging circuit 110 when the nozzle assembly 26 is removed, to reduce risk of exposure to the user when handling the housing 22 while the nozzle assembly 26 is detached. The nozzle assembly 26 can include an actuator 178 that closes the safety switch 176 when the nozzle assembly 26 is attached to the housing 22, and that releases or opens the safety switch 176 when the nozzle assembly 26 is removed. While the safety switch 176 is open and the charging circuit 110 is disabled, the motor 50 can remain operable and can still be activated by depressing the trigger 54a to pump liquid solution through the sprayer 10 (e.g., to flush the fluid passageways or perform other servicing or maintenance to the sprayer 10).

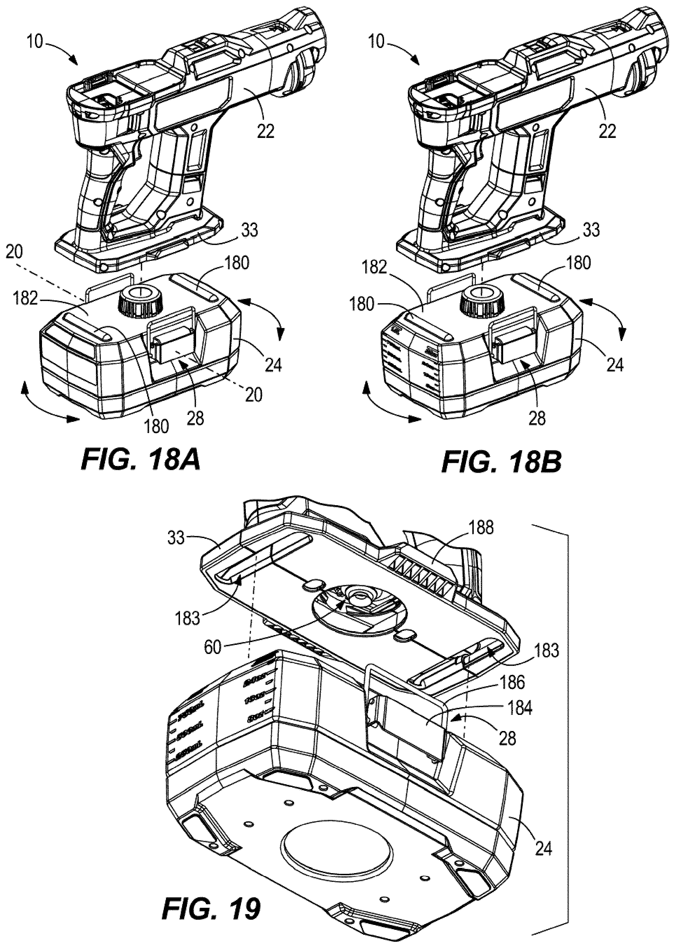

With reference to FIGS. 18A, 18B, and 19 the reservoir 24 is generally symmetrical about a centerline 20-20, and can be coupled to the reservoir attachment portion 33 in two different orientations 180 degrees apart. The reservoir 24 includes alignment ridges 180 provided in a top wall 182, such that when the reservoir 24 is coupled to the reservoir attachment portion 33, the alignment ridges 180 are received into alignment recesses 183 (FIG. 19) formed in the reservoir attachment portion 33 to ensure the reservoir 24 is adequately aligned therewith. The reservoir 24 also includes over-centered latches 28 including a latch cam 184 pivotably coupled to each respective side of the reservoir 24, and a latch buckle 186 pivotably coupled to each respective latch cam 184. The reservoir attachment portion 33 includes a pair of hooks 188 provided at opposite sides thereof to receive the latch buckles 186, and the latch cams 184 are pivoted downward to secure the reservoir 24 and exert a clamping force between the reservoir 24 and the reservoir attachment portion 33.

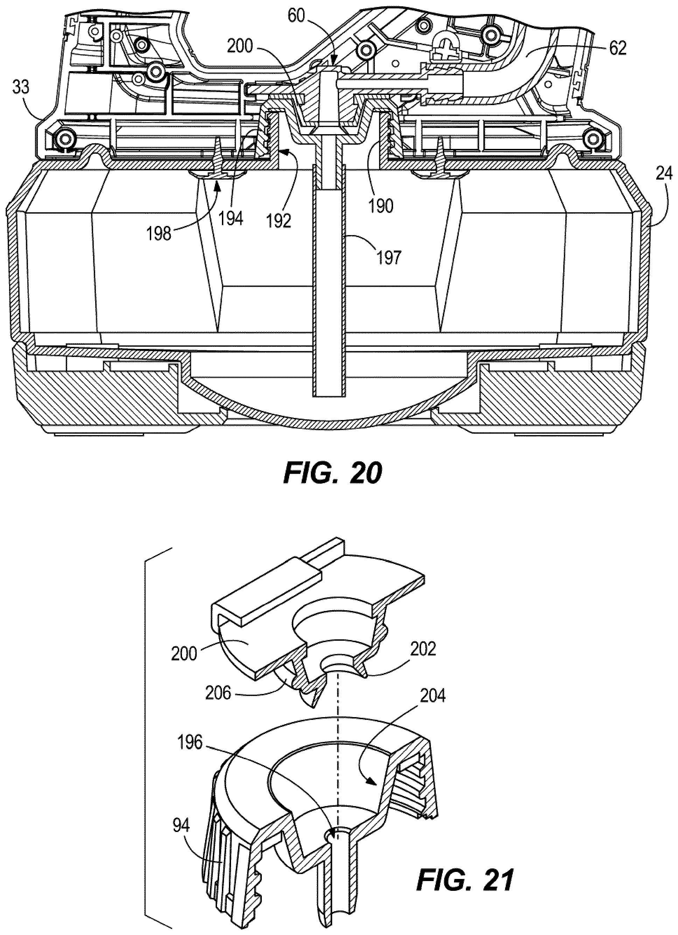

With reference to FIGS. 20 and 21, the reservoir 24 includes a neck portion 190 that defines a reservoir opening 192 for pouring liquid solution into the reservoir 24. The neck portion 190 is threaded and couples to a reservoir cap 194. The cap 194 defines a central cap opening 196 and couples to a pickup tube 197 that extends to a bottom region of the reservoir 24 to draw the liquid solution therefrom. The reservoir 24 also includes one or more check valves 198 provided in the upper wall of the reservoir 24 to permit a replacement volume of air to enter into the reservoir 24 as the liquid solution is drawn out of the reservoir 24 during operation of the sprayer 10. In the illustrated embodiment, the check valve 198 is provided as a single umbrella valve 198.

The sprayer 10 also includes the solution inlet 60 coupled to the inlet conduit 62 and that forms a liquid seal with the reservoir cap 194 to fluidly connect the reservoir 24 to the sprayer 10. The solution inlet 60 includes a rubber gasket 200 having a frustoconical surface 202 that forms a primary seal with a tapered recess 204 formed in the reservoir cap 194, and an integrally molded O-ring 206 that forms a secondary seal with the tapered recess 204. When the reservoir 24 is clamped to the reservoir attachment portion 33, the clamping force exerted by the latches 28 compresses the rubber gasket 200 between the solution inlet 60 and the reservoir cap 194 to ensure an adequate seal therebetween.

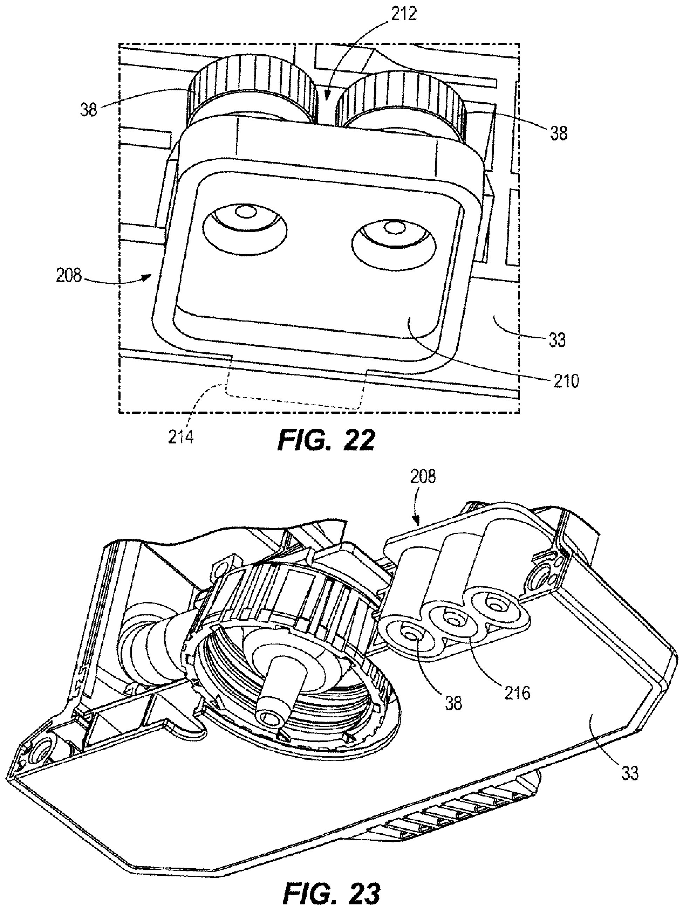

With reference to FIGS. 22 and 23, in some embodiments, the sprayer 10 can include nozzle storage receptacles 208 for storing additional nozzles 38 onboard the sprayer 10. In the embodiment shown in FIG. 22, the nozzle storage receptacle 208 includes a molded cover 210 that secures the nozzles 38 within a recess 212 formed in the reservoir attachment portion 33. The cover 210 includes a tab 214 for grasping and removing the cover 210 to access the nozzles 38. In the embodiment shown in FIG. 23, the nozzle storage receptacle 208 includes a plurality of molded tubes 216 supported within the reservoir attachment portion 33, and each molded tube 216 can receive a nozzle 38 for storing between the housing 22 and the reservoir 24.

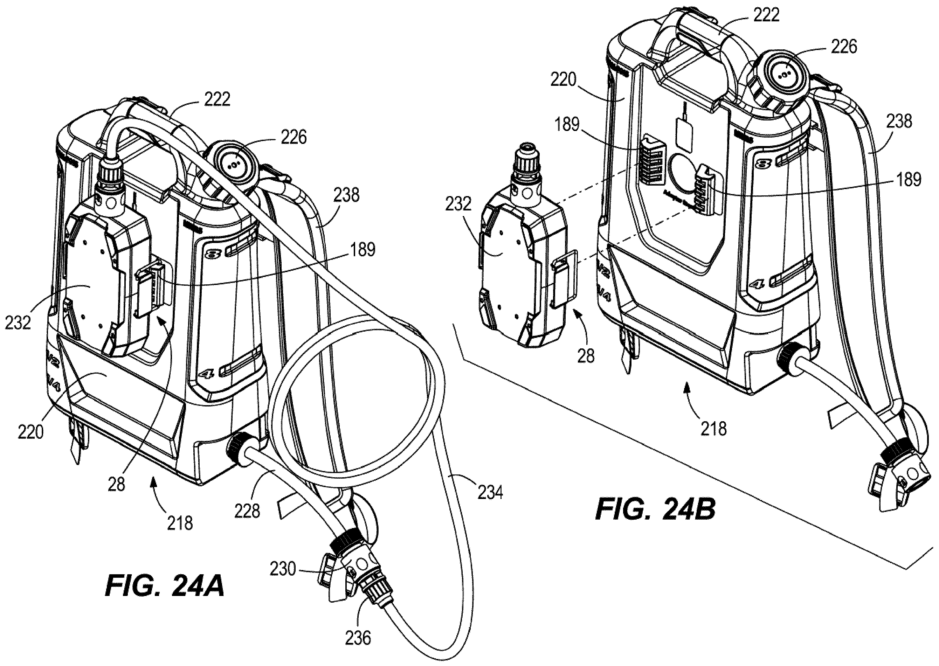

FIGS. 24A and 24B illustrate a remote reservoir 218 that can be used with the sprayer 10 in place of the onboard reservoir 24 to supply liquid solution to the sprayer 10. The remote reservoir 218 includes a tank 220 for storing the liquid solution, the remote reservoir 218 defining a remote reservoir handle 222 and a fill opening (not shown) covered by a tank cap 226. The remote reservoir 218 also includes a flexible hose 228 extending from a lower portion of the tank 220 and terminating with a first quick connect fitting 230. The remote reservoir 218 further includes an adapter 232 that removably attaches to the reservoir attachment portion 33 of the sprayer 10 to thereby fluidly connect the remote reservoir 218 to the sprayer 10. A hose 234 extends from the adapter 232 and terminates in a second quick connect fitting 236 that couples to the first quick connect fitting 230 of the flexible hose 228 to fluidly connect the adapter 232 to the tank 220. Like the onboard reservoir 24, the adapter 232 includes latches 28 that clamp to the hooks 188 on the reservoir attachment portion 33. The tank 220 also includes a pair of hooks 189. The latches 28 of the adapter 232 can also clamp to the hooks 189 formed on the tank 220 for storing the adapter 232 when the adapter 232 is not coupled to the sprayer 10.

In some embodiments, the remote reservoir 218 includes backpack straps 238 attached to the tank 220 such that the tank 220 may be worn by the user. In some embodiments the backpack straps 238 may be omitted and the tank 220 can be carried directly by the handle 222, or set down to rest upon a ground surface. In the same or other embodiments, the straps can be omitted, the tank 220 can be inserted into a cloth backpack for carrying by the user. In operation, the user fills the tank 220 with a liquid solution and couples the adapter 232 to the reservoir attachment portion 33. Then, the user operates the sprayer 10 in a manner similar to that described above with respect to the onboard reservoir 24.

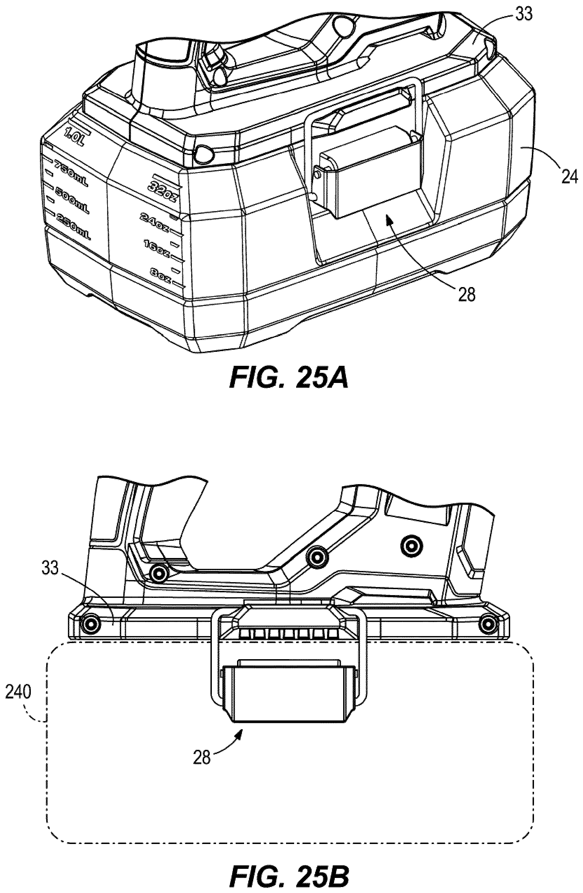

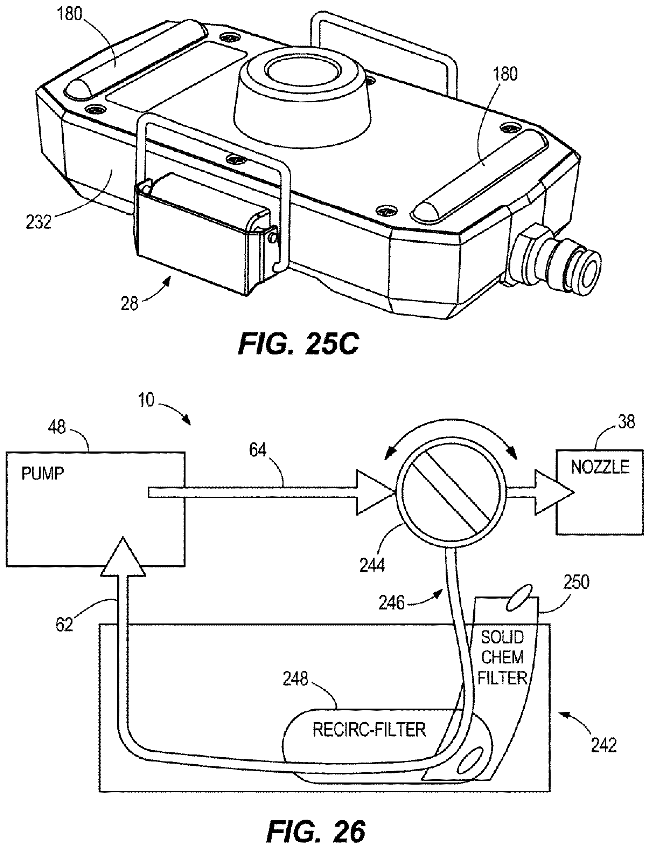

FIG. 25A illustrates the onboard reservoir 24 coupled to the reservoir attachment portion 33. The illustrated onboard reservoir 24 is a one-gallon reservoir 24 that can hold one gallon of liquid solution. FIG. 25B illustrates another embodiment of an onboard reservoir 240 configured as a two-gallon reservoir 240 that can hold two gallons of liquid solution. The reservoirs 24, 240 are interchangeable and can each be coupled to the reservoir attachment portion 33 in the manner described above. FIG. 25C illustrates the adapter 232 of the remote reservoir 218. Like the onboard reservoir 24 described above, the adapter 232 is reversible and can be coupled to the reservoir attachment portion 33 in two different orientations, and in the same manner as described above with respect to the onboard reservoir 24.

FIG. 26 schematically illustrates another embodiment of the sprayer 10 that includes a priming and recirculating system 242 for recirculating fluid between the pump 48 and the reservoir 24. In addition to the reservoir 24, the inlet conduit 62, the pump 48, and the outlet conduit 64, the priming and recirculating system 242 includes a valve 244 disposed between the outlet conduit 64 and the nozzle assembly 26, and a low restriction return line 246 extending between the valve 244 and the reservoir 24. The valve 244 is a two position, three-way valve 244 movable between a first or spray position, at which the outlet conduit 64 is fluidly connected to the nozzle assembly 26, and a second or recirculating position, at which the outlet conduit is fluidly connected to the return line 246. The valve 244 can be directly accessed and actuated by the user to switch between the spraying and recirculating positions. When the valve 244 is located in the recirculation position, the pump 48 can be operated to evacuate trapped air out of the fluid passageways of the sprayer 10 and direct the air back into the reservoir 24 via the return line 246. This allows the sprayer 10 to be primed much faster than by spraying the air through the nozzle 38, which can have high restrictions that may impede pumping when the air is in the system. Additionally, the priming and recirculating system 242 can include a recirculation filter 248 coupled to the return line 246 to filter debris or other contaminants from the liquid solution. Moreover, the recirculating system 242 can include a mesh container 250 that receives solid chemicals that may be dissolved to create the desired liquid solution inside the reservoir 24. By recirculating liquid solution through the priming and recirculating system 242, the solution inside the reservoir 24 can be stirred or agitated to facilitate dissolving the solid chemicals.

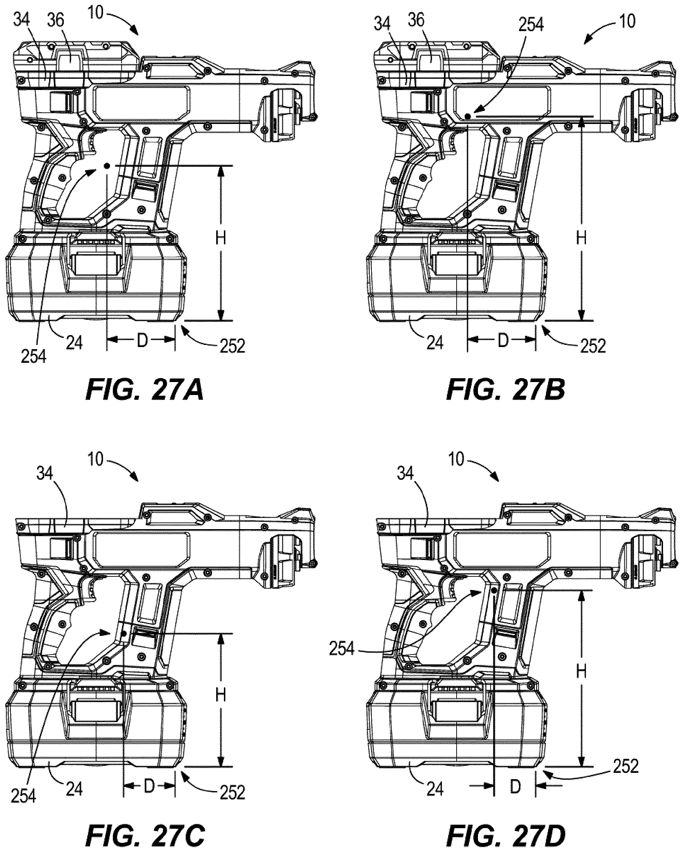

FIGS 27A-27D illustrate the sprayer 10 at a resting position oriented as if the reservoir 24 were resting on a flat horizontal surface (not shown). At the resting position, the battery receptacle 34 is located directly above the handle portion 32 and the reservoir attachment portion 33 is located below the handle portion 32. By locating the battery receptacle 34 directly above the handle portion 32, the center of gravity of the battery pack 36 is nearly centered over the handle portion 32 and a tilting moment about the handle portion 32 is reduced. Additionally, the openings formed in the battery pack 36 face downwards when the battery pack 36 is coupled to the battery receptacle 34, reducing the likelihood of liquid ingress into the battery pack 36 during operation of the sprayer 10. The center of gravity of the reservoir 24 is likewise located relatively close to the handle portion 32, thereby similarly reducing a tilting moment about the handle portion 32 exerted by the reservoir 24, as compared to traditional sprayers that locate the reservoir closer to the nozzle assembly.

A forward edge of the bottom of the reservoir 24 defines a tipping fulcrum 252 about which the sprayer 10 can rotate. In FIGS. 27A-27D, the sprayer 10 includes a center of gravity 254 located at a height H above the tipping fulcrum 252, and at a depth D behind the tipping fulcrum 252. FIG. 27A illustrates the sprayer 10 with the battery pack 36 coupled to the battery receptacle 34 and the one-gallon reservoir 24 filled with liquid solution. In this configuration, the height H is 168.75 millimeters (mm) and the depth D is 68.90 mm. FIG. 27B illustrates the sprayer 10 with the battery pack 36 coupled to the battery receptacle 34 and the one-gallon reservoir 24 empty. In this configuration, the height H is 220.31 mm and the depth D is 64.04 mm. FIG. 27C illustrates the sprayer 10 with the battery pack 36 removed from the battery receptacle 34 and the one-gallon reservoir 24 filled with liquid solution. In this configuration, the height H is 139.98 mm and the depth D is 56.65 mm. FIG. 27D illustrates the sprayer 10 with the battery pack 36 removed from the battery receptacle 34 and the one-gallon reservoir 24 empty. In this configuration, the height H is 193.74 mm and the depth D is 40.45 mm.

Various features of the disclosure are set forth in the following claims.

## Claims

1. A fluid sprayer comprising:
a housing defining a reservoir attachment portion;
a pump supported within the housing;
a nozzle assembly supported by the housing and located in fluid communication with the pump;
an electrostatic charging circuit configured to induce an electrostatic charge in a fluid pumped out of the nozzle assembly by the pump;
a first reservoir including a first latch configured to releasably secure the first reservoir to the reservoir attachment portion to fluidly couple the first reservoir to the pump; and
a second reservoir including a tank, an adapter, and a hose fluidly connecting the tank to the adapter, the adapter including a second latch configured to releasably secure the adapter to the reservoir attachment portion to fluidly couple the tank to the pump.

2. The fluid sprayer of claim 1, wherein the reservoir attachment portion defines a hook configured to engage the first latch and the second latch and/or
wherein each of the first latch and the second latch includes a latch cam and a latch buckle pivotably coupled to the latch cam.

3. The fluid sprayer of claim 1 or 2, wherein the first reservoir includes a third latch configured to releasably secure the first reservoir to the reservoir attachment portion, the first latch and the third latch being coupled to opposite respective sides of the first reservoir, and wherein the adapter includes a fourth latch configured to releasably secure the adapter to the reservoir attachment portion, the second latch and the fourth latch being coupled to opposite respective sides of the adapter and/or
wherein the reservoir attachment portion defines a first hook configured to engage each of the first latch, the second latch, the third latch, and the fourth latch, and the reservoir attachment portion further defines a second hook configured to engage each of the first latch, the second latch, the third latch, and the fourth latch.

4. The fluid sprayer of one of claims 1 to 3, wherein the nozzle assembly includes a removable nozzle, and wherein the reservoir attachment portion defines a nozzle receptacle configured to receive and hold the removable nozzle and/or
wherein the first reservoir covers the nozzle receptacle in response to the first reservoir being secured to the reservoir attachment portion.

5. The fluid sprayer of one of claims 1 to 4, wherein:
the first reservoir includes a neck portion defining a neck opening, and a cap configured to couple to the neck portion, the cap including a central cap opening,
the sprayer includes an inlet fluidly connected to the pump, the inlet including a gasket, and
the gasket seals against the cap to fluidly connect the reservoir to the pump in response to the first reservoir being secured to the reservoir attachment portion and/or
wherein the first latch is configured to exert a clamping force to compress the gasket against the cap.

6. The fluid sprayer of claim 5, wherein the cap includes a tapered recess defined about the central cap opening, and wherein the gasket includes a frustoconical surface that seals against a surface of the tapered recess in response to the first reservoir being secured to the reservoir attachment portion and/or
wherein the tank includes a pair of tank hooks configured to engage the first latch and the second latch to selectively secure the adapter to the tank and/or
wherein the second reservoir includes carry straps coupled to the tank.

7. A fluid sprayer comprising:
a housing defining a reservoir attachment portion having an alignment recess;
a pump supported within the housing;
a nozzle assembly supported by the housing and located in fluid communication with the pump;
an electrostatic charging circuit configured to induce an electrostatic charge in a fluid pumped out of the nozzle assembly by the pump;
a first reservoir configured to releasably secure to the reservoir attachment portion and including a first alignment ridge configured to be received into the alignment recess to align the first reservoir with the reservoir attachment portion; and
a second reservoir including a tank, an adapter configured to releasably secure to the reservoir attachment portion to fluidly couple the tank to the pump, and a hose fluidly connecting the tank to the adapter, the adapter including a second alignment ridge configured to be received into the alignment recess to align the adapter with the reservoir attachment portion.

8. The fluid sprayer of claim 7, wherein:
the alignment recess comprises a first alignment recess and the reservoir attachment portion further includes a second alignment recess,
the first reservoir includes a neck portion defining a neck opening, and a cap configured to couple to the neck portion, the cap including a central cap opening,
the first reservoir further includes a third alignment ridge located opposite the first alignment ridge with respect to the neck portion,
the first alignment ridge is configured to be received into the first alignment recess and the third alignment ridge is configured to be received into the second alignment recess.

9. The fluid sprayer of claim 8, wherein the second alignment ridge of the adapter is configured to be received into the first alignment recess, and the adapter includes a fourth alignment ridge configured to be received into the second alignment recess.

10. The fluid sprayer of one of claims 7 to 9, wherein the reservoir attachment portion defines a hook, the first reservoir includes a first latch configured to releasably engage the hook to releasably secure the first reservoir to the reservoir attachment portion, and the adapter includes a second latch configured to releasably engage the hook to releasably secure the adapter to the reservoir attachment portion and/or
wherein each of the first latch and the second latch includes a latch cam and a latch buckle pivotably coupled to the latch cam.

11. The fluid sprayer of one of claims 7 to 10, wherein the nozzle assembly includes a removable nozzle, and wherein the reservoir attachment portion defines a nozzle receptacle configured to receive and hold the removable nozzle, and wherein the first reservoir covers the nozzle receptacle in response to the first reservoir being secured to the reservoir attachment portion, and wherein the adapter covers the nozzle receptacle in response to the adapter being secured to the reservoir attachment portion.

12. The fluid sprayer of one of claims 7 to 11, wherein:
the first reservoir includes a neck portion defining a neck opening, and a cap configured to couple to the neck portion, the cap including a central cap opening and a tapered recess defined about the central cap opening,
the sprayer includes an inlet fluidly connected to the pump, the inlet including a gasket having a frustoconical surface that seals against a surface of the tapered recess to fluidly connect the first reservoir to the pump in response to the first reservoir being secured to the reservoir attachment portion.

13. A fluid sprayer comprising:
a housing defining a reservoir attachment portion;
a pump supported within the housing;
a nozzle assembly supported by the housing and located in fluid communication with the pump;
an electrostatic charging circuit configured to induce an electrostatic charge in a fluid pumped out of the nozzle assembly by the pump;
a first reservoir configured to releasably secure to the reservoir attachment portion to fluidly couple the first reservoir to the pump, the first reservoir including a first alignment ridge configured to align the first reservoir in a first orientation relative to the reservoir attachment portion and in a second orientation relative to the reservoir attachment portion, the second orientation being one hundred eighty degrees away from the first orientation; and
a second reservoir including a tank, an adapter configured to releasably secure to the reservoir attachment portion to fluidly couple the tank to the pump, and a hose fluidly connecting the tank to the adapter, the adapter including a second alignment ridge configured to align the adapter in a third orientation relative to the reservoir attachment portion and in a fourth orientation relative to the reservoir attachment portion, the third orientation being one hundred eighty degrees away from the fourth orientation.

14. The fluid sprayer of claim 13, wherein:
the reservoir attachment portion includes a first alignment recess and a second alignment recess,
the first alignment ridge is received into the first alignment recess in response to the first reservoir being secured to the reservoir attachment portion in the first orientation,
the first alignment ridge is received into the second alignment recess in response to the first reservoir being secured to the reservoir attachment portion in the second orientation,
the second alignment ridge is received into the first alignment recess in response to the adapter being secured to the reservoir attachment portion in the third orientation,
the second alignment ridge is received into the second alignment recess in response to the adapter being secured to the reservoir attachment portion in the fourth orientation.

15. The fluid sprayer of claim 13 or 14, wherein the reservoir attachment portion defines a hook, the first reservoir includes a first latch configured to releasably engage the hook to releasably secure the first reservoir to the reservoir attachment portion, and the adapter includes a second latch configured to releasably engage the hook to releasably secure the adapter to the reservoir attachment portion.
